# EUROPEAN PATENT SPECIFICATION

(11) **EP 0 658 332 B1**
(45) Date of publication and mention of the grant of the patent: **29.09.1999**
(21) Application number: 94203557.7
(22) Date of filing: 07.12.1994
(51) Int. Cl.: A61B 6/10, A61B 6/00

(54) **X-ray examination apparatus comprising a balanced X-ray detector**
Strahlendetektor mit Gewichtsausgleich für Röntgen-Untersuchungsgerät
Appareil d'examen par radiographie comprenant un détecteur à contrepoids

(30) Priority: 15.12.1993 BE 9301396
(43) Date of publication of application: 21.06.1995
(73) Proprietor: Koninklijke Philips Electronics N.V., 5621 BA Eindhoven (NL)
(72) Inventor: van der Heijden, Pieter Antonius, NL-5656 AA Eindhoven (NL); Janssen, Jozef Theodorus Antonius, NL-5656 AA Eindhoven (NL); van Vilsteren, Carmen Francisca Maria, NL-5656 AA Eindhoven (NL)
(74) Representative: Bakker, Hendrik

(56) References cited:
- DE-A- 3 343 924
- DE-C- 736 294
- GB-A- 1 057 557
- GB-A- 2 085 627
- US-A- 4 961 214
- US-A- 5 105 455

## Description

The invention relates to an X-ray examination apparatus, comprising a frame and a support which is connected thereto and which supports at a first end an X-ray source and at a second end an X-ray detector which is provided with a housing, said X-ray source and X-ray detector being arranged opposite one another in such a manner that a central ray of an X-ray beam emitted by the X-ray source is approximately coincident with a connecting line between the X-ray source and the X-ray detector, the X-ray detector being movable along the connecting line, and at least a part of the outer surface of the housing of the X-ray detector which is situated near its end facing the X-ray source, encloses such an angle relative to the connecting line that the distance between this part and the connecting line increases as the distance from the X-ray source increases,

An example of such an apparatus is known from US patent No. 5,105,455.

Generally, known X-ray examination apparatus provide combined rotation of an X-ray source and an X-ray detector, secured at opposite ends of f.i. a C-shaped support, about two mutually perpendicular axes. These axes may intersect at a point (the isocentre) which is situated on the connecting line between the X-ray source and the X-ray detector. Generally speaking, such support can also be moved in the vertical direction relative to the frame. Such apparatus is suitable for irradiating an object from different directions, the X-ray source and the X-ray detector being arranged so as to face one another and the direction of irradiation being adjustable. In order to prevent detrimental effects of collisions between the object to be examined (for example, a patient positioned on a patient table) and the X-ray detector known apparatus may comprise collision protection devices which deactivate the support drive when the X-ray detector approaches the object to be examined or another object.

The apparatus as described in the said US patent is provided with an X-ray detector that has a collision protection device consisting of a protection cover at the periphery, formed of an elastic material such as rubber, and carries a pressure sensitive detection mechanism containing optical fibers. When the X-ray detector collides with an object, the elastic cover material deforms and elastically deforms one or more optical fibers, thereby disturbing light flowing through the fibers. The disturbance is detected by an optical sensor which interrupts a driver to stop further movement of the X-ray detector. It should be clear that movement of the X-ray detector can be stopped in response to a sensing of pressure on the protective cover only if there is some mechanism that responds to such detection which can mechanically control the moving detector. Apparently, in this known apparatus the mechanical position of the X-ray detector is determined by a drive, which is enabled to change the positions of the X-ray detector only during absence of pressure on the protective cover.

Such devices must be capable of detecting the approaching very accurately and reliably. Therefore, they are comparatively expensive and complex.

It is an object of the invention to improve the known X-ray examination apparatus so that for almost any collision with another object, the X-ray detector will be pushed away from this object by the force of the collision, regardless of the direction of approach of the object, the force exerted on the object being so small that the risk of damage is minimized. To achieve this, the X-ray examination apparatus in accordance with the invention is characterized in that the X-ray detector is manually movable along the said connecting line and a shift of the centre of gravity of the X-ray detector occurring during said movement being substantially compensated for by a movement of a counterweight which is coupled to the movement of the X-ray detector, so that movement of the X-ray detector requires only a comparatively small force in a direction parallel to the connecting line,
said part of the outer surface of the housing of the X-ray detector being sufficiently rigid such that whenever said part collides with an object, a component of force on the X-ray detector in a direction parallel to the connecting line is sufficient for deflecting the X-ray detector along the connecting line and away from the object.

The invention is based on the idea that the force exerted on a surface extending at an angle relative to the connecting line upon a collision will virtually always contain a component which is directed along the connecting line. This component will be directed away from the X-ray source if the distance between the surface and the connecting line increases as the distance from the X-ray source increases. As a result, the X-ray detector will be pushed away from the X-ray source and hence also from the object with which the collision took place. Therefore, the collision will not cause any damage and a separate device for the prevention of collisions is not required in the X-ray examination apparatus in accordance with the invention. The magnitude of the component of the force which is produced by the collision and which is directed along the connecting line is dependent inter alia on the angle enclosed by the connecting line relative to said part of the outer surface. The other circumstances remaining the same, this component will be greater as said angle is larger.

As the X-ray detector is manually movable along the connecting line and is balanced by way of the counterweight, the distance between the X-ray source and the X-ray detector can be readily changed manually, so that different magnifications can be adjusted for the X-ray image to be formed. It is a further advantage of the balancing of the X-ray detector that in given cases detrimental effects of a collision between the object to be examined and the X-ray detector are prevented. For example, when the support is in a position such that the connecting line between the X-ray source and the X-ray detector extends substantially vertically and the support is then moved in the vertical direction in such a manner that the X-ray detector collides with the object, the X-ray detector will be pushed away from the object by the force of the collision. Consequently, the collision will not damage the object or the X-ray detector. However, if the X-ray detector does not approach the object along the connecting line, the force of the collision also acts in a direction other than the direction of the connecting line, so that the X-ray detector will very likely not be pushed away from the object. In that case the collision could have serious consequences for the object (the patient).

Unlike in the known apparatus, the present invention does not rely upon mechanical resiliency of a cover on the X-ray detector housing or a pressure sensitive mechanism within the cover to protect against damage or injury when the X-ray detector collides with an object. Indeed, in the present invention the X-ray detector is not even controlled in position (at least not along the connecting line between the X-ray detector and the X-ray source) by an electromechanical device. It should be apparent that a pressure sensor cannot stop movement of the X-ray detector in a direction in which mechanical position is being manually rather than electromechanically controlled, at least not without adding some kind of braking mechanism controlled by the pressure sensor. In accordance with this invention, the X-ray detector is not only manually controlled and counterweighted in the direction that connects the X-ray source and the X-ray detector, but also the outer surface of the housing of the X-ray detector is tapered and sufficiently rigid so that when there is a collision of the tapered part of the X-ray detector with an object, a component of force along the direction that connects the X-ray detector and X-ray source is always generated that deflects the X-ray detector away from the object in this direction.

A preferred embodiment of the apparatus in accordance with the invention is characterized in that at its end which faces the X-ray source the housing of the X-ray detector is shaped approximately as a truncated cone. For the effect of the oblique part of the outer surface of this embodiment it is irrelevant at which point of the housing the collision actually takes place.

In most cases the operator will stop drive means causing the support to move relative to an object to be examined as soon as a collision occurs. In the exceptional case where this does not happen, the collision could cause damage as yet when the X-ray detector reaches its extreme position at the second end of the support. This is because the X-ray detector cannot move further backwards in that case. In order to prevent damage also in these cases, a further preferred embodiment of the apparatus in accordance with the invention is characterized in that there is provided a detection element which, when the X-ray detector is situated at a maximum distance from the X-ray source, is operative to supply a signal which deactivates the drive means for the period during which a force component which is directed away from the X-ray source acts on the X-ray detector in the direction of the connecting line.

These and other aspects of the invention will be described in detail hereinafter with reference to the drawing.
Fig. 1 is a side elevation of an embodiment of an X-ray examination apparatus in accordance with the invention,
Fig. 2 shows a feasible embodiment of a part of the apparatus shown in Fig. 1, and
Fig. 3 shows a further detail.

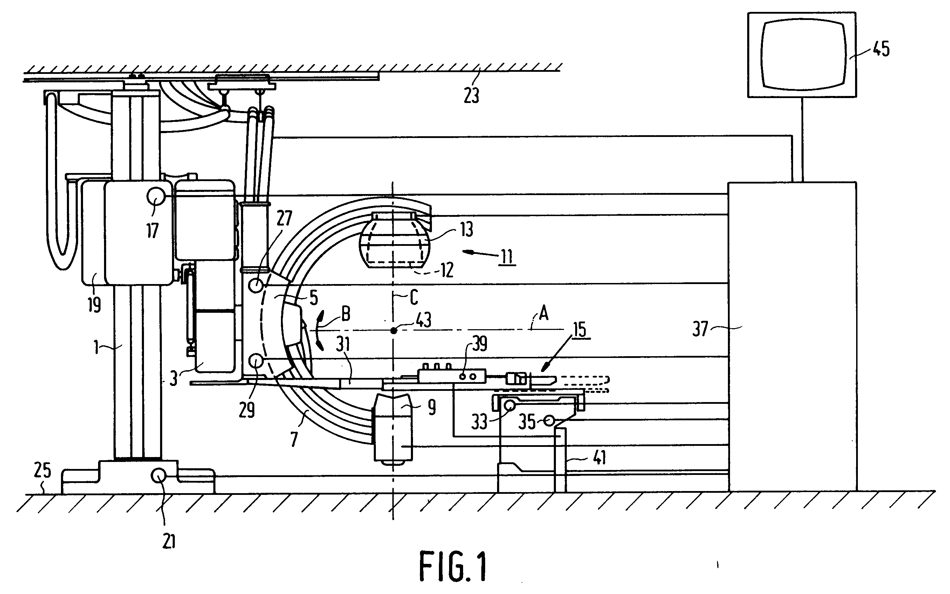

Fig. 1 shows an X-ray examination apparatus, comprising a frame with a vertical column 1 and an arm 3 whereto a support 7 is connected via a holder 5. In the present embodiment the support 7 is shaped as a C-arm having a first end whereto an X-ray source 9 is secured and a second end whereto an X-ray detector 11 is secured. Evidently, supports of other shape, for example a U-shaped arm, are also feasible. The X-ray detector 11 comprises (denoted by dashed lines) an X-ray image intensifier tube 12 which is accommodated in a housing 13. An object to be examined, for example a patient (not shown), can be arranged on a table 15 between the X-ray source 9 and the X-ray detector 11. A frame portion 19 supporting the arm 3 can be displaced in the vertical direction along the column 1 by means of a motor 17. A motor 21 enables displacement of the column 1 in the horizontal direction on rails provided in a ceiling 23 and a floor 25. The holder 5 in which the C-arm 7 is secured and in which the arm is displaceable in the circumferential direction of the C-arm by means of a motor 27, is rotated about an axis A when driven by a motor 29. The displacement of the C-arm 7 in the circumferential direction is denoted by the double arrow B. The table 15 comprises a top 31 which is displaceable in the horizontal direction by means of a motor 33 and in the vertical direction by means of a motor 35. Said motors, constituting drive means for the support 7 and the table 15, are controlled by a control unit 37 which comprises a computer supplying control signals. The user of the X-ray examination apparatus can apply instructions to the control unit 37, via an interface 41, by operating controls on a control unit 39 which is mounted on the table 15 in known manner. In dependence on these instructions, the control unit 37 applies control signals to a given motor, to the X-ray source 9 or to the X-ray detector 11.

The X-ray source 9 and the X-ray detector 11 are arranged opposite one another in such a manner that a central ray of a beam of X-rays emitted by the X-ray source is approximately coincident with the connecting line C between the X-ray source and the X-ray detector. The axis A and the connecting line C intersect at a point 43 which is referred to as the isocentre. A part of a patient, arranged on the table 15, which is situated in the isocentre 43 is always imaged in the same location on an exit screen of the X-ray image intensifier tube 12 by an X-ray beam emitted by the X-ray source 9. The image formed can be studied by means of a monitor 45.

The X-ray examination apparatus described thus far is known per se. The invention can also be used in other X-ray examination apparatus which are also known per se and in which, for example a frame of different construction is present or movable parts are driven in a different manner.

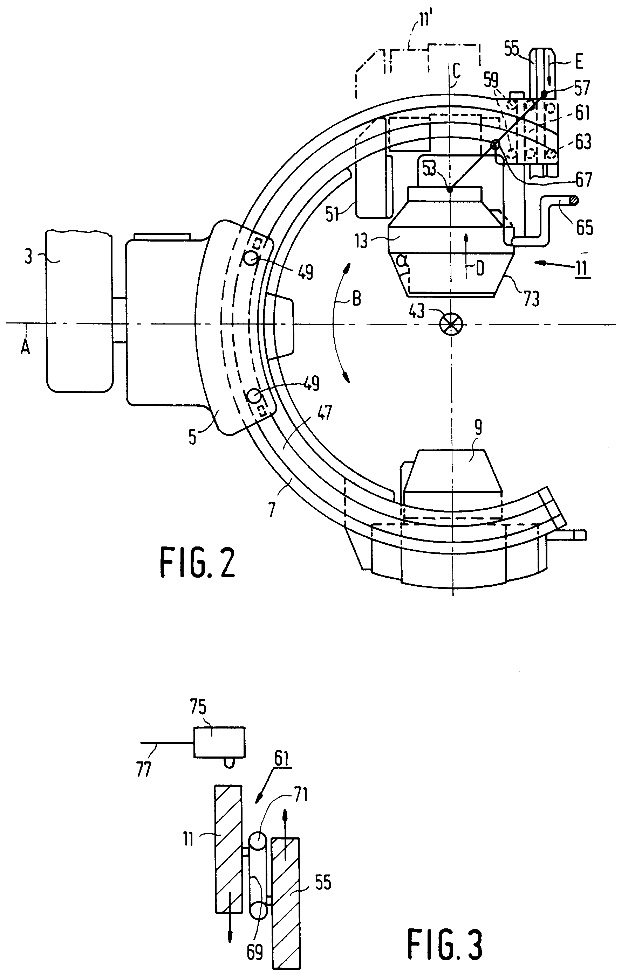

Fig. 2 is a more detailed representation of a feasible embodiment of the support 7 whereto the X-ray source 9 and the X-ray detector 11 are secured. The support 7 is provided on both sides with slits 47 (only one of which is visible in the Figure), which receive bearing wheels 49 enabling the movability of the support according to the double arrow B. The X-ray detector 11 converts an X-ray image of an object arranged between the X-ray source 9 and the X-ray detector into an optical image which can be picked up by a video camera 51 so as to be displayed by the monitor 45 (Fig. 1). The X-ray detector 11 has a centre of gravity in a position 53 and a counterweight 55 has a centre of gravity in a position 57. A motion of the X-ray detector 11 parallel to the connecting line C is guided by the wheels 59 and transferred to the counterweight 55 in the opposite direction by transmission means 61. The movement of the counterweight 55 is guided by wheels 63. When the X-ray detector 11 is moved in the direction of the arrow D, for example by means of a grip 65 connected thereto, the counterweight 55 coupled to the X-ray detector moves in the direction of the arrow E. As a result, the common centre of gravity of the X-ray detector 11 and the counterweight 55 remains in a position 67. Thus, the shift of the centre of gravity of the X-ray detector 11 is compensated for by the shift of the centre of gravity of the counterweight 55. Therefore, movement of the X-ray detector 11 in the direction of the connecting line C requires a force in the direction of the connecting line which is only very small relative to the mass of the X-ray detector.

Fig. 3 is a diagrammatic representation of a feasible embodiment of the coupling between the movements of the X-ray detector 11 and the counterweight 55 which are shown as blocks for the sake of simplicity of the Figure. The transmission means 61 are formed by a drive belt or chain 69 which forms a closed loop which is guided about two (possibly toothed) guide wheels 71. The X-ray detector 11 and the counterweight 55 are both connected to the drive belt or chain 69, so that the movement of the X-ray detector is transferred to the counterweight in the opposite direction.

As is clearly shown in Fig. 2, the end of the housing 13 of the X-ray detector 11 which faces the X-ray source 9 is shaped approximately as a truncated cone. The part 73 of the outer surface of the housing 13 which is situated near this end encloses an angle α of, for example 30° relative to the connecting line C, so that the distance between this part of the outer surface and the connecting line increases as the distance from the X-ray source 9 increases. When the X-ray detector 11 is in a comparatively low position (i.e. comparatively close to the X-ray source 9), as shown in Fig. 2, and when in this position the table 15 and the support 7 are moved, for example horizontally relative to one another by means of said drive motors, it may occur that a patient arranged on the table collides with the X-ray detector. In almost all cases the part 73 of the outer surface of the housing 13 which is situated near the X-ray source 9 will then come into contact with the patient. Thanks to the slanted position of this part of the outer surface, the force released by this collision will always have a component which is directed away from the X-ray source 9 in the direction of the connecting line C. As a result, the X-ray detector 11 will be moved in the direction of the arrow D, so that it moves away from the patient. Therefore, the collision will not have any adverse effects on the patient or the X-ray detector 11.

In the described embodiment the part 73 of the outer surface of the housing 13 which is situated near the Xray source 9 is shaped as a truncated cone. Thanks to this shape, any movement including a horizontal component upon collision will cause a movement of the X-ray detector 11 in the direction of the arrow D. If the X-ray examination apparatus is designed so that movement of the X-ray detector relative to the table 15 can take place in one direction only, for example parallel to the axis A, it suffices when only the faces of the relevant part of the outer surface of the housing 13 which extend in this direction enclose an angle a relative to the connecting line C. In Fig. 2 these are the faces extending approximately perpendicularly to the plane of drawing. The other faces may be parallel to the connecting line C. It is also to be noted that said value of the angle α (30°) is rather arbitrary. Generally speaking, the force component in the direction of the connecting line C is greater as the value of the angle is greater. Values other than said value, for example 45° or 60°, therefore, will offer better results in many cases.

The movement of the X-ray detector 11 in the direction of the arrow D is limited by a stop (not shown). This stop defines an extreme position of the X-ray detector 11 which is denoted by dash-dot lines 11' in Fig. 2. Should a collision take place between the X-ray detector 11 and an object to be examined while the X-ray detector 11 is in the extreme position, or should the X-ray detector reach the extreme position due to such a collision without deactivation of the drive motors by the operator, the collision could still have detrimental effects. In order to prevent this, a detection element 75 as diagrammatically shown in Fig. 3 is preferably provided near the second end of the support 7. The detection element 75 may be formed, for example by a switch or a known optical or magnetic sensor. The detection element 75 is connected to the control unit 37 (Fig. 1) by means of a lead 77. When the X-ray detector 11 is in the extreme position (at a maximum distance from the X-ray source 9), the detection element 75 supplies the control unit 37 with a signal which switches off the drive motors for as long as a force component acts in the direction of the connecting line C due to a collision with an object.

## Claims

1. An X-ray examination apparatus, comprising a frame (1, 3) and a support (7) which is connected thereto and which supports at a first end an X-ray source (9) and at a second end an X-ray detector (11) which is provided with a housing (13), said X-ray source and X-ray detector being arranged opposite one another in such a manner that a central ray of an X-ray beam emitted by the X-ray source is approximately coincident with a connecting line (C) between the X-ray source and the X-ray detector and at least a part (73) of the outer surface of the housing (13) of the X-ray detector (11) which is situated near its end facing the X-ray source (9), encloses such an angle (α) relative to the connecting line (C) that the distance between this part and the connecting line increases as the distance from the X-ray source increases,
characterized in that
the X-ray detector is manually movable along the said connecting line (C) and a shift of the centre of gravity of the X-ray detector occurring during said movement being substantially compensated for by a movement of a counterweight (55) which is coupled to the movement of the X-ray detector, so that movement of the X-ray detector requires only a comparatively small force in a direction parallel to the connecting line,
said part (73) of the outer surface of the housing (13) of the X-ray detector (11) being sufficiently rigid such that whenever said part (73) collides with an object, a component of force on the X-ray detector in a direction parallel to the connecting line is sufficient for deflecting the X-ray detector along the connecting line and away from the object.

2. An X-ray examination apparatus as claimed in Claim 1, characterized in that at its end which faces the X-ray source (9) the housing (13) of the X-ray detector (11) is shaped approximately as a truncated cone.

3. An X-ray examination apparatus as claimed in Claim 1 or 2, provided with drive means (17, 21, 27, 29, 33, 35) for moving the support (7) and an object to be examined relative to one another, characterized in that there is provided a detection element (75) which, when the X-ray detector (11) is situated at a maximum distance from the X-ray source (9), is operative to supply a signal which deactivates the drive means (17, 21, 27, 29, 33, 35) for the period during which a force component which is directed away from the X-ray source acts on the X-ray detector in the direction of the connecting line (C).

## Patentansprüche

1. Röntgenuntersuchungsgerät, mit einem Gestell (1, 3) und einem daran befestigten Träger (7), der an einem ersten Ende eine Röntgenquelle (9) und an einem zweiten Ende einen Röntgendetektor (11) trägt, der mit einem Gehäuse (13) versehen ist, wobei die genannte Röntgenquelle und der Röntgendetektor einander gegenüber so angeordnet sind, dass ein von der Röntgenquelle emittierter zentraler Strahl eines Röntgenstrahlenbündels ungefähr mit einer Verbindungslinie (C) zwischen der Röntgenquelle und dem Röntgendetektor zusammenfällt und zumindest ein Teil (73) der Außenfläche des Gehäuses (13) des Röntgendetektors (11), der nahe dessen der Röntgenquelle (9) zugewandten Ende liegt, einen solchen Winkel (α) mit der Verbindungslinie (C) bildet, dass der Abstand zwischen diesem Teil und der Verbindungslinie zunimmt, wenn der Abstand von der Röntgenquelle aus zunimmt,
dadurch gekennzeichnet, dass
der Röntgendetektor entlang der genannten Verbindungslinie (C) mit der Hand bewegt werden kann und eine bei dieser Bewegung auftretende Verschiebung des Schwerpunktes des Röntgendetektors nahezu durch eine Bewegung eines Gegengewichts (55) kompensiert wird, das mit der Bewegung des Röntgendetektors gekoppelt ist, so dass das Bewegen des Röntgendetektors nur eine verhältnismäßig kleine Kraft in einer Richtung parallel zur Richtung der Verbindungslinie erfordert,
wobei der genannte Teil (73) der Außenfläche des Gehäuses (13) des Röntgendetektors (11) genügend starr ist, so dass immer, wenn der genannte Teil (73) mit einem Objekt kollidiert, eine auf den Röntgendetektor wirkende Kraftkomponente in einer Richtung parallel zur Verbindungslinie ausreichend ist, um den Röntgendetektor entlang der Verbindungslinie und weg von dem Objekt abzulenken.

2. Röntgenuntersuchungsgerät nach Anspruch 1, dadurch gekennzeichnet, dass das Gehäuse (13) des Röntgendetektors (11) an seinem der Röntgenquelle (9) zugewandten Ende ungefähr wie ein abgeschnittener Kegel geformt ist.

3. Röntgenuntersuchungsgerät nach Anspruch 1 oder 2, mit Antriebsmitteln (17, 21, 27, 29, 33, 35), um den Träger (7) und ein zu untersuchendes Objekt relativ zueinander zu bewegen, dadurch gekennzeichnet, dass ein Detektionselement (75) vorgesehen ist, das, wenn der Röntgendetektor (11) sich auf maximalem Abstand von der Röntgenquelle (9) befindet, fähig ist, ein Signal abzugeben, das die Antriebsmittel (17, 21, 27, 29, 33, 35) während der Zeitdauer deaktiviert, in der eine von der Röntgenquelle weg gerichtete Kraftkomponente in Richtung der Verbindungslinie (C) auf den Röntgendetektor wirkt.

## Revendications

1. Appareil d'examen radiographique, comprenant un cadre (1, 3) et un support (7) qui lui est relié et qui supporte, à une première extrémité, une source de rayons X (9) et, à une deuxième extrémité, un détecteur de rayons X (11) qui est muni d'un logement (13), ladite source de rayons X et ledit détecteur de rayons X étant agencés l'un en face de l'autre, de manière à ce qu'un rayon central d'un faisceau de rayons X émis par la source de rayons X coïncide approximativement avec une ligne de liaison (C) entre la source de rayon X et le détecteur de rayons X, et à ce qu'au moins une partie (73) de la surface extérieure du logement (13) du détecteur de rayons X (11), située à proximité de son extrémité faisant face à la source de rayons X (9), comprenne un angle (α) par rapport à la ligne de liaison (C) tel que la distance entre cette partie et la ligne de liaison augmente à mesure que la distance par rapport à la source de rayons X augmente,
caractérisé en ce que
le détecteur de rayons X peut être déplacé manuellement le long de ladite ligne de liaison (C), un décalage du centre de gravité du détecteur de rayons X se produisant pendant ledit déplacement étant en grande partie compensé par un déplacement d'un contrepoids (55) qui est couplé au déplacement du détecteur de rayons X, de sorte que le déplacement du détecteur de rayons X nécessite seulement une force relativement faible dans une direction parallèle à la ligne de liaison,
ladite partie (73) de la surface extérieure du logement (13) du détecteur de rayons X (11) étant suffisamment rigide pour que, chaque fois que ladite partie (73) rencontre un objet, une composante de force sur le détecteur de rayons X dans une direction parallèle à la ligne de liaison soit suffisante pour dévier le détecteur de rayons X le long de la ligne de liaison et à l'opposé de l'objet.

2. Appareil d'examen radiographique suivant la revendication 1, caractérisé en ce qu'à son extrémité faisant face à la source de rayons X (9), le logement (13) du détecteur de rayons X (11) a approximativement la forme d'un tronc de cône.

3. Appareil d'examen radiographique suivant la revendication 1 ou 2, muni de moyens d'entraînement (17, 21, 27, 29, 33, 35) pour déplacer le support (7) et un objet à examiner l'un par rapport à l'autre, caractérisé en ce qu'il est prévu un élément de détection (75) qui, lorsque le détecteur de rayons X (11) est situé à une distance maximale de la source de rayons X (9), fonctionne pour fournir un signal qui désactive les moyens d'entraînement (17, 21, 27, 29, 33, 35) au cours de la période pendant laquelle une composante de force, qui est dirigée à l'opposé de la source de rayons X, agit sur le détecteur de rayons X dans la direction de la ligne de liaison (C).
